(19) Europäisches Patentamt
European Patent Office
Office européen des brevets

(11) Veröffentlichungsnummer: **0 011 107**
**B1**

(12) # EUROPÄISCHE PATENTSCHRIFT

(45) Veröffentlichungstag der Patentschrift: **22.06.83**

(51) Int. Cl.³: **G 01 N 33/52,**
G 01 N 31/22

(21) Anmeldenummer: **79103568.6**

(22) Anmeldetag: **21.09.79**

(54) **Methode zur Bestimmung einer Amino- oder Aminosulfonsäure.**

(30) Priorität: **10.11.78 CH 11589/78**

(43) Veröffentlichungstag der Anmeldung:
**28.05.80 Patentblatt 80/11**

(45) Bekanntmachung des Hinweises auf die Patenterteilung:
**22.06.83 Patentblatt 83/25**

(84) Benannte Vertragsstaaten:
**CH DE FR GB IT NL**

(56) Entgegenhaltungen:
**DE - A - 2 263 534**
**FR - A - 2 134 850**
**FR - A - 2 204 303**
**FR - A - 2 221 728**

**Die Akte enthält technische Angaben, die nach dem Eingang der Anmeldung eingereicht wurden und die nicht in dieser Patentschrift enthalten sind.**

(73) Patentinhaber: **F. HOFFMANN-LA ROCHE & CO. Aktiengesellschaft**
**CH-4002 Basel (CH)**

(72) Erfinder: **Gillessen, Dieter, Dr.**
**Oberfeldstrasse 12**
**CH-4133 Pratteln (CH)**
Erfinder: **Lergier, William**
**Liebrütistrasse 42**
**CH-4303 Kaiseraugst (CH)**

(74) Vertreter: **Kloter, Rudolf et al,**
**Grenzacherstrasse 124 Postfach 601**
**CH-4002 Basel (CH)**

(56) Entgegenhaltungen:
**CHEMICAL ABSTRACTS, Band 89, Nr. 3. 17. Juli 1978, Zusammenfassung Nr. 19514e, Seite 278, Columbus, Ohio, US, D. J. REEDER et al.: "o-Phthalaldehyde for the fluorometric assay of nonprotein amino compounds"**
**CHEMICAL ABSTRACTS, Band 82, Nr. 22, 2. Juni 1975, Zusammenfassung Nr. 149032n, Seite 631, Columbus, Ohio, US, J. R. BENSON et al.: "o-Phthalaldehyde. Fluorogenic detection of primary amines in the picomole range"**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 011 107 B1

Methode zur Bestimmung einer Amino- oder Aminosulfonsäure

Die vorliegende Erfindung betrifft eine Bestimmungsmethode für eine aromatische Amino- oder Aminosulfonsäure der allgemeinen Formel

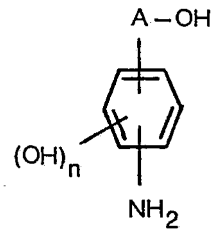

I

worin A —CO— oder —$SO_2$— und n 0,1 oder 2 bedeutet, in einer Urin-, Plasma- oder Serumprobe.

Gewisse Peptide enthaltend einen Amino- oder Aminosulfonsäurerest der allgemeinen Formel

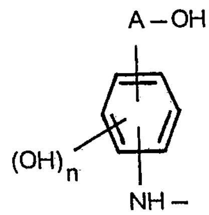

I'

worin A und n die genannte Bedeutung haben, werden als diagnostische Mittel zur Bestimmung der Pankreasfunktion eingesetzt. Ein für diesen Zweck besonders geeignetes Peptid ist die N-Benzoyl-L-tyrosyl-p-aminobenzoesäure der Formel

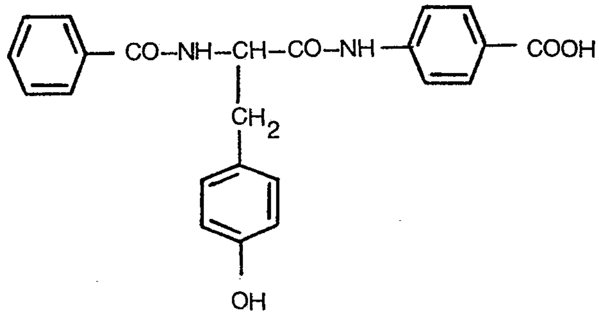

II

Diese Pankreasfunktionsprüfung wird im Prinzip wie folgt durchgeführt:

Einem nüchternen Probanden, bei dem vorgängig allfällige Medikamente, insbesondere Sulfonamide, Sulfonylharnstoffe und Pankreasfermente abgesetzt worden sind, wird eine ausreichende Menge eines geeigneten Peptides enthaltend einen Amino- oder Aminosulfonsäurerest der Formel I' verabreicht. Durch Trinkenlassen von Wasser wird für eine genügende Diurese gesorgt. Durch das vom Pankreas produzierte Chymotrypsin wird das erwähnte Peptid gespalten, worauf ein grosser Teil der Amino- oder Aminosulfonsäure der Formel I als solche oder in metabolisierter Form im Urin ausgeschieden wird. Zur Rückgewinnung allfällig metabolisierter Amino- oder Aminosulfonsäuren der Formel I wird der Urin vorgängig der Bestimmung zweckmässigerweise hydrolysiert. Je nach der prozentualen Menge an in der Urinprobe gefundener Amino- oder Aminosulfonsäure der Formel I kann auf die Funktion des Pankreas geschlossen werden. Sofern als Peptid die N-Benzoyl-L-tyrosyl-p-aminobenzoesäure verwendet wird bestimmt man in der Urinprobe als Aminosäure der Formel I die p-Aminobenzoesäure. Die Bestimmung der Amino- oder Aminosulfonsäure der Formel I kann aber auch in Plasma oder Serum erfolgen.

Bei der bekannten Bestimmungsmethode nach Bratton und Marshall modifiziert nach Smith et al. in J. Uin. Inrest. 24,388—404 (1945), wird die in der Urinprobe vorhandene Amino- oder Aminosulfonsäure der Formel I diazotiert und zu einem Farbstoff gekuppelt, welcher dann colorimetrisch bestimmt wird. Da diese Bestimmungsmethode relativ aufwendig ist, besteht ein Bedürfnis nach einer einfacheren, weniger komplizierten Bestimmungsmethode.

Im Rahmen der vorliegenden Erfindung wurde nun gefunden, dass man die im Urin oder im eiweissfreien Plasma oder Serum enthaltene Amino- oder Aminosulfonsäure der Formel I durch Umsetzen derselben mit 4-Phenyl-spiro[furan-2(3H)1'-phthalan]-3,3'-dion (Fluram$^{R}$), mit 2-Methoxy-2,4-diphenyl-3(2H)-furanon oder mit o-Phthaldialdehyd bei einem pH Bereich von 1,5—3 und

gegebenenfalls in Gegenwart eines inerten organischen Lösungsmittels umsetzt und den erhaltenen Komplex mittels einer photometrischen Messung bestimmt.

Die vorliegende Erfindung betrifft demnach eine Bestimmungsmethode für eine Amino- oder Aminosulfonsäure der Formel I in Urin, Plasma oder Serum, welche dadurch gekennzeichnet, ist, dass man eine gegebenenfalls mit Wasser verdünnte Urinprobe oder eine eiweissfreie Plasma- oder Serumprobe bei einem pH Bereich von 1,5—3 und gegebenenfalls in Gegenwart eines inerten Organischen Lösungsmittels mit 4-Phenyl-spiro[furan-2(3H)1′-phthalan]-3,3′-dion, mit 2-Methoxy-2,4-diphenyl-3(2H)-furanon oder mit o-Phthaldialdehyd umsetzt und anschliessend eine photometrische Messung durchführt.

4-Phenyl-spiro[furan-2(3H)-phthalan]-3,3′-dion ist aus der DE—A 2 263 534 bekannt. 2-Methoxy-2,4-diphenyl-3(2H)-furanon ist aus der DE—A 2 407 899 bekannt.

Bei Verwendung von 4-Phenyl-spiro[furan-2(3H)1′-phthalan]-3,3′-dion ist die Anwesenheit eines inerten organischen Lösungsmittels erforderlich. Als Lösungsmittel sind Aceton oder Aethanol bevorzugt. Auch bei Verwendung von 2-Methoxy-2,4-diphenyl-3(2H)-furanon ist die Anwesenheit eines inerten organischen Lösungsmittels erforderlich. Bei der Verwendung von o-Phthaldialdehyd wird vorzugsweise Aethanol als Lösungsmittel und Mercaptoäthanol als Reduktionsmittel verwendet. Der für die Reaktion optimale pH-Bereich liegt bei ca. 2—2,5. Dieser wird dadurch erreicht, dass die nach der Hydrolyse zu saure Urinprobe mit Wasser verdünnt oder abgepuffert wird. Als Puffer wird vorzugsweise ein Na-Acetatpuffer oder ein Kaliumchlorid/Salzsäure-puffer verwendet.

Die nachfolgenden Beispiele erläutern die Erfindung.

Beispiel 1

a) Bestimmungsmethode

1 ml unverdünnter Urin und 0,1 ml 10 N Salzsäure werden in einem verschlossenen Reagensglas während einer Stunde bei 96±2°C im Wasserbad erhitzt.

0.1 ml des hydrolysierten Urins, 5 ml Natriumacetatpuffer und 1 ml Fluram$^R$-Reagens werden gut gemischt und 15 Minuten stehen gelassen. Nach 15 Minuten werden Analysenproben entnommen und die Extinktionen werden bei 400 nm (405 nm bei Filtergeräten) gegen den Blindwert (Messansatz wie oben, aber mit 0,1 ml Wasser anstelle von hydrolysiertem (Urin) gemessen.

b) Herstellung des Natriumacetatpuffers

27,21 g Natriumacetat-Trihydrat werden in ca. 500—700 ml Wasser bidest. gelöst, worauf unter Umrühren und Zutropfen von Eisessig das pH auf 3 gestellt wird. Nach Zugabe von 3 Tropfen pHix-Konservierungsmittel wird mit Wasser auf 1000 ml aufgefüllt. Man erhält einen 0,2 m Natriumacetatpuffer von pH 3. Anstelle dieses Puffers kann auch der nachfolgend beschriebene verwendet werden:

2,72 g Natriumacetat werden in ca. 900 ml Wasser bidest. gelöst, worauf durch Zugaben von ca. 55 ml Eisessig das pH auf 3 eingestellt wird. Man verdünnt mit Wasser auf 1000 ml und erhält einen 0,02 m Natriumacetatpuffer.

c) Herstellung von Fluram$^R$-Reagens

140 mg 4-Phenylspiro[furan-2(3H)1′-phthalan]-3,3′-dion (Fluram$^R$) werden in 100 ml Aceton gelöst.

Herstellung einer Eichkurve

a) Stammlösung

Ca. 40 mg p-Aminobenzoesäure werden in 30 ml Aceton gelöst, worauf mit Natriumacetatpuffer auf 100 ml aufgefüllt wird.

b) Verdünnungen

Ca. 8 μg/0,1 ml=2 ml Stammlösung+8 ml Natriumacetatpuffer
ca. 16 μg/0,1 ml=4 ml Stammlösung+6 ml Natriumacetatpuffer
ca. 20 μg/0,1 ml=5ml Stammlösung+5 ml Natriumacetatpuffer
ca. 24 μg/0,1 ml=6 ml Stammlösung+4 ml Natriumacetatpuffer
ca. 32 μg/0,1 ml=8 ml Stammlösung+2 ml Natriumacetatpuffer
ca. 40 μg/0,1 ml=unverdünnt

Die Extinktionen werden bei 400 nm (405 nm bei Filtergeräten) analog wie die Urinproben gemessen und die gemessenen Werte werden auf Millimeterpapier gezeichnet. Man erhält eine lineare Eichkurve.

Beispiel 2

Bestimmungsmethode

Es wird wie in Beispiel 1 gearbeitet, jedoch unter Verwendung von 2-Methoxy-2,4-diphenyl-3(2H)-furanon anstelle von 4-Phenylspiro[furan-2(3H)1′-phthalan]-3,3′-dion. Die Extinktionen werden bei 320 nm gemessen.

Eichkurve

Man erhält eine lineare Eichkurve im Bereich von 40—200 $\mu$g p-Aminobenzoesäure pro Messansatz (ca. 3—6 ml).

Beispiel 3

Bestimmungsmethode

Es wird analog wie in Beispiel 1 gearbeitet jedoch wird anstelle von Fluram$^{R}$-Reagens o-Phthaldialdehyd in Aethanol enthaltend 0,5% Mercaptoäthanol verwendet. Die Extinktionen werden bei 420 nm gemessen. Man erhält eine lineare Eichkurve im Bereich von 8—80 $\mu$g p-Aminobenzoesäure pro Messansatz (ca. 3—6 ml).

Beispiel 4

Es wird wie in Beispiel 1 gearbeitet, jedoch unter Verwendung von 1 ml eiweissfreiem Plasma oder Serum. Die Enteiweissung kann nach an sich bekannten Methoden erfolgen. Man erhält eine lineare Eichkurve im Bereich von 2—35 $\mu$g p-Aminobenzoesäure pro Messansatz wie in Beispiel 1.

**Patentansprüche**

1. Methode zur Bestimmung einer Amino- oder Aminosulfonsäure der allgemeinen Formel

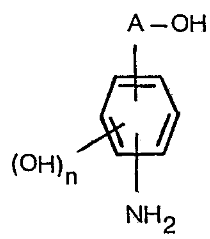

I

worin A —CO— oder —$SO_2$— und n 0,1 oder 2 bedeutet, in Urin, Plasma oder Serum, welche dadurch gekennzeichnet, ist, dass man eine gegebenenfalls mit Wasser verdünnte Urinprobe oder eine eiweissfreie Plasma- oder Serumprobe bei einem pH-Bereich von pH 1,5—3 und gegebenenfalls in Gegenwart eines inerten, organischen Lösungsmittels mit 4-Phenylspiro[furan-2(3H)1′-phthalan]-3,3′-dion, mit 2 Methoxy-2,4-diphenyl-3(2H)-furanon oder mit o-Phthaldialdehyd umsetzt und anschliessend eine photometrische Messung durchführt.

2. Methode nach Anspruch 1, dadurch gekennzeichnet, dass die Urin-, Plasma- oder Serumprobe vorgängig der Bestimmung hydrolysiert wird.

3. Methode nach Anspruch 1, dadurch gekennzeichnet, dass man mit 4-Phenyl-spiro[furan-2(3H)-1′-phthalan]-3,3′-dion arbeitet und dass man als Lösungsmittel Aceton verwendet.

4. Methode nach Anspruch 3, dadurch gekennzeichnet, dass man als Lösungsmittel Aethanol verwendet.

5. Methode nach einem der Ansprüche 1 bis 4, dadurch gekennzeichnet, dass man in einem pH-Bereich von 2—2,5 arbeitet.

6. Methode nach Anspruch 5, dadurch gekennzeichnet, dass man in einem Puffersystem arbeitet.

7. Methode nach Anspruch 6, dadurch gekennzeichnet, dass man als Puffer einen Natriumacetatpuffer verwendet.

8. Methode nach Anspruch 6, dadurch gekennzeichnet, dass man als Puffer einen Kaliumchloride/Salzsäure-puffer verwendet.

**Revendications**

1. Méthode de détermination d'un acide aminé ou aminosulfonique de formule générale

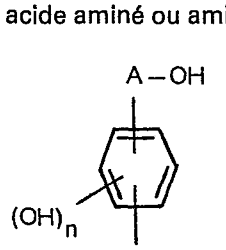

I

dans laquelle A représente —CO— ou —$SO_2$— et n est égal à 0, 1 ou 2, dans l'urine, le plasma ou le sérum, caractérisée en ce que l'on fait réagir un échantillon d'urine éventuellement dilué par l'eau ou un échantillon de plasma ou de sérum exempt de protéines dans un intervalle de pH allant de pH 1,5 à

3 et le cas échéant en présence d'un solvant organique inerte avec la 4-phényl-spiro[furanne-2(3H)1'-phtalane]-3,3'-dione, la 2-méthoxy-2,4-diphényl-3(2H)-furannone ou le dialdéhyde o-phtalique et on procède ensuite à une mesure photométrique.

2. Méthode selon la revendication 1, caractérisée en ce que l'échantillon d'urine, de plasma ou de sérum est hydrolysé avant la détermination.

3. Méthode selon la revendication 1, caractérisée en ce que l'on opère avec la 4-phényl-spiro-[furanne-2(3H)-1'-phtalane]-3,3'-dione et en ce que l'on utilise l'acétone comme solvant.

4. Méthode selon la revendication 3, caractérisée en ce que l'on utilise l'éthanol comme solvant.

5. Méthode selon l'une des revendications 1 à 4, caractérisée en ce que l'on opère dans un intervalle de pH de 2 à 2,5.

6. Méthode selon la revendication 5, caractérisée en ce que l'on opère dans un système tampon.

7. Méthode selon la revendication 6, caractérisée en ce que l'on utilise en tant que tampon un tampon à l'acétate de sodium.

8. Méthode selon la revendication 6, caractérisée en ce que l'on utilise en tant que tampon un tampon chlorure de potassium/acide chlorhydrique.

**Claims**

1. Method for the determination of an amino or amino-sulphonic acid of the general formula

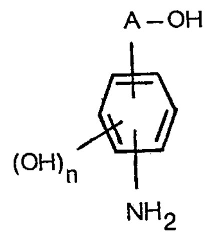

I

wherein A signifies —CO— or —$SO_2$— and n signifies 0, 1 or 2, in urine, plasma or serum, which is characterized by reacting a urine sample, which is optionally diluted with water, or an albumin-free plasma or serum sample in a pH range of pH 1.5—3 and, if necessary, in the presence of an inert, organic solvent with 4-phenyl-spiro[furan-2(3H)1'-phthalan]-3,3'-dione, with 2-methoxy-2,4-diphenyl-3(2H)-furanone or with o-phthaldialdehyde and subsequently carrying out a photometric measurement.

2. Method according to claim 1, characterized in that the urine, plasma or serum sample is hydrolyzed prior to the determination.

3. Method according to claim 1, characterized in that it is carried out with 4-phenyl-spiro[furan-2(3H)1'-phthalan]-3,3'-dione and in that acetone is used as the solvent.

4. Method according to claim 3, characterized in that ethanol is used as the solvent.

5. Method according to any one of claims 1 to 4, characterized in that it is carried out in a pH range of 2—2.5

6. Method according to claim 5, characterized in that it is carried out in a buffer system.

7. Method according to claim 6, characterized in that a sodium acetate buffer is used as the buffer.

8. Method according to claim 6, characterized in that a potassium chloride/hydrochloric acid buffer is used as the buffer.